# EUROPEAN PATENT APPLICATION

(11) **EP 4 847 132 A1**
(43) Date of publication of application: **02.12.2026**
(21) Application number: 24919275.8
(22) Date of filing: 24.01.2024
(51) Int. Cl.: B01L 3/00, B81C 1/00, B82Y 5/00, B82Y 40/00

(54) **HIGH-THROUGHPUT MICRO-NANO DEVICE BASED ON ELECTROMECHANICAL SYNERGISTIC EFFECT AND PREPARATION METHOD THEREFOR AND USE THEREOF**

(71) Applicant: Shenzhen Institutes of Advanced Technology, Chinese Academy of Sciences, Shenzhen, Guangdong 518055 (CN)
(72) Inventor: YANG, Hui, Shenzhen, Guangdong 518055 (CN); HAO, Rui, Shenzhen, Guangdong 518055 (CN); ZHANG, Yi, Shenzhen, Guangdong 518055 (CN)
(74) Representative: Gao, Lin
(86) International application number: PCT/CN2024/073868
(87) International publication number: WO 2025/156160

(57) **Abstract**

A fluid control device having a micro-nano structure and an electrode structure includes a nanochannel array and a microchannel array. The microchannel array includes an inlet microchannel array and an outlet microchannel array; the inlet microchannel array includes at least one inlet microchannel, and the outlet microchannel array includes at least one outlet microchannel; the nanochannel array includes at least one nanochannel; electrodes are disposed adjacent to the nanochannel; and the thickness of the electrode is 80%-110% of the height of the nanochannel. The device efficiently achieves poration of biological particles and loading of exogenous substances into the same by synergistically applying an electric field and mechanical stimulation.

## Description

### TECHNICAL FIELD

The present disclosure pertains to the field of micro-nanofluidics, and in particular, relates to a high-throughput micro-nano device based on an electromechanical synergistic effect, a fabrication method, and use thereof.

### BACKGROUND

Extracellular vesicles are nanoparticles secreted by various cells and having a phospholipid bilayer structure, with a diameter of about 30 nm-2000 nm. As communication mediators or carriers between cells, extracellular vesicles can achieve the transfer and transport of substances such as lipids, nucleic acids, proteins, and enzymes. Compared with conventional nanocarrier systems such as nanoparticles, liposomes, and viruses, extracellular vesicles are regarded as an emerging natural nanocarrier due to their advantages including low immunogenicity, long circulation time, low organ toxicity, good biocompatibility, and strong tissue penetration capability. Therefore, constructing a novel nano-delivery system based on extracellular vesicles has important theoretical significance and practical value for experimental research and clinical applications.

To achieve loading of exogenous substances into extracellular vesicles, one of the most commonly used methods is electroporation. The basic loading process includes uniformly mixing extracellular vesicles and exogenous substances, adding the mixture into an electroporation cuvette, and then placing the electroporation cuvette into an electroporator. High-voltage stimulation is applied to the extracellular vesicles through the electroporator, triggering phospholipid molecules on the membrane surface to shift and form temporary pores, allowing exogenous substance molecules to enter the interior of the extracellular vesicles through the pores. Following the application of the electric field, the phospholipid molecules on the membrane surface rearrange and restore to an intact state, thereby achieving loading of exogenous substances into extracellular vesicles. Although this loading method can effectively improve the loading efficiency of extracellular vesicles using an electric field, the conventional electroporation method mainly relies on bulky conventional equipment, and sample processing units with large characteristic dimensions result in excessively high applied voltages, and different extracellular vesicles are liable to be subjected to non-uniform electric field stimulation, which not only may interfere with or destroy the exogenous substances, but even may damage the integrity and biological activity of the extracellular vesicles. Therefore, it is necessary to develop a safer and more efficient loading method with a uniform and controllable physical field.

Micro-nanofluidic technology, due to its characteristic dimensions being similar to the dimensions of extracellular vesicles, has the capability of precisely manipulating extracellular vesicles at the micro-nano scale, and therefore has exhibited tremendous application potential in research fields such as extracellular vesicle separation, extracellular vesicle detection, extracellular vesicle preparation, and extracellular vesicle loading. Therefore, to achieve precise processing of extracellular vesicles, Chinese Patent Publication No. CN110975953A discloses a micro-nanofluidic chip and a fabrication method and use thereof. Since the height of the constricted nanochannel of the chip is similar to the dimensions of extracellular vesicles, a mechanical squeezing effect can be applied to the extracellular vesicles, thereby achieving loading of exogenous substances. However, this chip only applies a certain mechanical squeezing effect during the extracellular vesicle loading process, resulting in a single physical stimulation with very limited efficacy. In addition, extracellular vesicles also exhibit severe size heterogeneity. For extracellular vesicles having dimensions smaller than the height of the nanochannel, the fixed-size micro-nanofluidic chip applies a relatively weak mechanical squeezing effect, thereby resulting in failure of small-sized extracellular vesicles to effectively load exogenous substances.

The above prior art has the following technical defects.

Firstly, the solutions in the prior art employ only one of the electroporation methods or the mechanoporation methods for achieving loading of exogenous substances into extracellular vesicles. Such a single loading mode results in a lack of synergistic effects of multiple physical fields, and the effect is very limited, thereby resulting in relatively low loading efficiency of extracellular vesicles.

Secondly, existing electroporation methods have the limitation that the feature sizes of the core processing unit are excessively large, resulting in non-uniform electric field stimulation on extracellular vesicles. In addition, a relatively high applied voltage may also cause problems such as aggregation of exogenous substances, and may even damage the integrity and biological activity of extracellular vesicles.

Thirdly, existing mechanoporation methods suffer from fixed nanochannel dimensions. Consequently, extracellular vesicles having dimensions smaller than the height of the nanochannel cannot be effectively processed, resulting in some small-sized extracellular vesicles being subjected to insufficient mechanical compression, thereby failing to load exogenous substances.

Fourthly, the micro-nanofluidics chip relied on by the existing mechanoporation methods only integrates, in its interior, a microchannel array and a nanochannel array, and does not integrate a micro-nano electrode array on a large scale inside the micro-nano channel arrays, resulting in a lack of possibility for synergistic manipulation by multiple physical fields in applications of extracellular vesicle loading in the prior art.

Lastly, existing micro-nano devices generally have the problem of low throughput, and only a small number of extracellular vesicles can be processed within a short period of time, making it difficult to satisfy the requirements of animal experiments and clinical applications.

### SUMMARY

To solve the problems in the prior art, a fluid control device having a micro-nano structure and an electrode array is provided. The device utilizes the synergy of electrical and mechanical stimulation to achieve reversible membrane poration of biological particles and loading of exogenous substances thereinto.

A first aspect of the present disclosure provides a fluid control device having a micro-nano structure and an electrode structure, and the fluid control device comprises a microchannel array, a nanochannel array, and an electrode array;
the microchannel array includes an inlet microchannel array and an outlet microchannel array; the inlet microchannel array includes at least one inlet microchannel, and the outlet microchannel array includes at least one outlet microchannel; the nanochannel array includes at least one nanochannel;
the inlet microchannel array and the outlet microchannel array are in fluid communication only through the nanochannel array, and a micro-nano channel network is formed in which inflow occurs only via an inlet and outflow occurs only via an outlet;
the inlet microchannel array is in fluid communication with the inlet, and the outlet microchannel array is in fluid communication with the outlet;
the electrode array includes a first electrode array and a second electrode array, the first electrode array includes at least one first electrode, the second electrode array at least includes one second electrode, and the first electrode and the second electrode are arranged in pairs and configured to form a substantially uniform electric field between the first electrode and the second electrode;
the first electrode array is electrically connected to a first node, and the second electrode array is electrically connected to a second node; the first node and the second node are each electrically connected to a power supply; and
the nanochannel is configured such that its width is greater than or equal to the particle size of target biological particles; and its height is 20%-200% of said particle size of the target biological particles; the first electrode and the second electrode are disposed within or adjacent to the nanochannel; the thickness of the first electrode and the thickness of the second electrode are 80%-110% of the height of the nanochannel; the first electrode and the second electrode are configured to provide a substantially uniform electric field for the target biological particles passing through the nanochannel.

In the technical solutions of the present disclosure, the cross-sectional shape of the nanochannel is square, rectangular, trapezoidal, circular, or triangular.

In the technical solutions of the present disclosure, the nanochannel is linear, serpentine, arcuate, wavy, spiral, or circular.

In the technical solutions of the present disclosure, the electrode array is disposed within the nanochannel, and each nanochannel includes at least one pair of the first electrode and the second electrode; the first electrode and the second electrode are disposed adjacent to and/or within the nanochannel along a fluid flow direction within the nanochannel.

In some technical solutions of the present disclosure, the first electrode array and the second electrode array respectively comprise a plurality of first electrodes and a plurality of second electrodes, and the first electrode array and the second electrode array are disposed in an interdigitated parallel configuration.

In the technical solutions of the present disclosure, the microchannel array and the nanochannel array are respectively disposed on different substrate layers, or are disposed on the same substrate layer.

In the technical solutions of the present disclosure, the microchannel array and the nanochannel array are respectively disposed on different substrate layers or the same substrate layer, and the microchannel array and the nanochannel array are alternately stacked.

In the technical solutions of the present disclosure, the microchannel array and the nanochannel array are respectively disposed on different substrate layers or the same substrate layer, and a substrate comprising the microchannel array includes at least one layer, preferably 1 layer, 2 layers, or 3 layers.

In the technical solutions of the present disclosure, the inlet microchannel array and the outlet microchannel array are disposed on the same substrate layer or disposed on different substrate layers.

In the technical solutions of the present disclosure, the inlet and the outlet are disposed on the same substrate layer or disposed on different substrate layers; and the inlet and the outlet are respectively disposed in a substrate including the microchannel array or disposed in a substrate including the nanochannel array.

In the technical solutions of the present disclosure, when the number of the inlet microchannels or the outlet microchannels is greater than 1, the depths and the widths thereof may be the same or different.

In the technical solutions of the present disclosure, the biological particles include biological particles having a particle size at the micrometer or nanometer scale, and the particle size ranges from 30 nm to 2000 nm. For example, the particle size may be 30 nm, 50 nm, 100 nm, 200 nm, 300 nm, 400 nm, 500 nm, 600 nm, 700 nm, 800 nm, 900 nm, 1000 nm, 1200 nm, 1400 nm, 1600 nm, 1800 nm, or 2000 nm.

In the technical solutions of the present disclosure, the nanochannel is configured such that its width is equal to or greater than the particle size of the target biological particles, and its height is less than, equal to, or greater than the particle size of the target biological particles.

In the technical solutions of the present disclosure, the height of the nanochannel may be 50%, 60%, 70%, 80%, 90%, 100%, 110%, or 120% of the particle size of the target biological particles; and the thickness of the electrode may be 90%, 95%, 96%, 97%, 98%, 99%, or 100% of the height of the nanochannel.

In the technical solutions of the present disclosure, the width of the nanochannel ranges from 1 to 10,000 times the particle size of the target biological particles, for example, it may be 20 times, 30 times, 50 times, 100 times, 200 times, 500 times, 1000 times, 2000 times, 5000 times, or 10,000 times.

In the technical solutions of the present disclosure, the microchannel array and the nanochannel array are respectively disposed on different substrate layers or the same substrate layer, and in the layer comprising the microchannel array, the inlet microchannel array and the outlet microchannel array are not in fluid communication with each other.

In the technical solutions of the present disclosure, the microchannel array and the nanochannel array are respectively disposed on different substrate layers or the same substrate layer, and the microchannel array and the nanochannel array are arranged at an angle greater than 0° and less than or equal to 90° with respect to each other.

In the technical solutions of the present disclosure, the number of the nanochannels in the nanochannel array layer between the inlet microchannel and the outlet microchannel is one or more, for example, 2, 3, 4, 5, 6, 7, 8, 9, 10, or more than 10.

In the technical solutions of the present disclosure, the heights of different nanochannels may be the same or different, and the widths of different nanochannels may be the same or different.

In the technical solutions of the present disclosure, the cross-sectional width and depth of the inlet microchannel and the outlet microchannel range from 30 nm to 1000 µm.

In the technical solutions of the present disclosure, the lengths of the first electrode and the second electrode are greater than or equal to the length of the nanochannel.

In the technical solutions of the present disclosure, the width of the first electrode or the second electrode is greater than or equal to 1 µm, preferably ranging from 1 µm to 100 µm.

In the technical solutions of the present disclosure, the distance between the first electrode and the second electrode is equal to the width of the nanochannel, preferably ranging from 30 nm to 1000 µm.

In the technical solutions of the present disclosure, the first electrode, the second electrode, and the substrate together constitute the nanochannel.

In the technical solutions of the present disclosure, the first electrode is disposed within or on one side of the nanochannel, and the second electrode is disposed within or on the other side of the nanochannel. Further, electrodes disposed within the nanochannel comprise first electrodes and second electrodes arranged in an interdigitated and staggered manner along a fluid flow direction within the nanochannel; a plurality of at least one of the first and second electrodes is provided, and a passage allowing target biological particles to pass therethrough is formed between the staggered first electrodes and second electrodes; and the distance between an adjacent first electrode and second electrode ranges from 30 nm to 1000 µm.

In the technical solutions of the present disclosure, the power supply is preferably a direct current power supply or an alternating current power supply.

In the technical solutions of the present disclosure, the substrate material for fabricating the nanochannel array is a micro-nano fabrication material, preferably glass, quartz, silicon wafer, silicon nitride wafer, silicon dioxide wafer, silicon carbide wafer, polydimethylsiloxane, polymethyl methacrylate, parylene C, polycarbonate, cyclic olefin copolymer, cyclic olefin polymer, polypropylene, photocurable resin, or soft thermoplastic elastomer, and the substrate material for fabricating a layer of the microchannel array is a micro-nano fabrication material, preferably silicon wafer, polydimethylsiloxane, silicon nitride wafer, silicon dioxide wafer, silicon carbide wafer, glass, quartz, polymethyl methacrylate, parylene C, polycarbonate, cyclic olefin copolymer, cyclic olefin polymer, polypropylene, photocurable resin, or soft thermoplastic elastomer.

In the technical solutions of the present disclosure, the inlet microchannel array and the outlet microchannel array are disposed in the same microchannel array layer or in different microchannel array layers.

In the technical solutions of the present disclosure, the electrode is deposited on a substrate by a thin film growth method, and further, the thin film growth method includes sputtering, evaporation, chemical vapor deposition, epitaxial growth, electrochemical deposition, inkjet printing, screen printing, chemical self-assembly, nanoimprint lithography, 3D printing, and two-photon polymerization printing.

In the technical solutions of the present disclosure, the electrode is made of a conductive material, such as gold, chromium, titanium, platinum, silver, copper, aluminum, indium tin oxide, polymer material, or nanomaterial.

In the technical solutions of the present disclosure, the method for fabricating the electrode includes the following steps:
i) forming grooves on a first substrate, where a depth of each groove is the height of the nanochannel;
ii) providing an electrode sacrificial layer on a surface of the first substrate, the electrode sacrificial layer having a shape complementary to the intended shape of the electrode;
iii) depositing an electrode layer on the first substrate coated with the electrode sacrificial layer, and removing the electrode sacrificial layer along with a portion of the electrode layer thereon; and obtaining pairs of electrodes disposed within or adjacent to the grooves formed in step i) and extending along an extending direction of the nanochannel, and
iv) setting a thickness of the electrode to be 80%-110% of the height of the nanochannel.

In the technical solutions of the present disclosure, in step iv), the thickness of the electrode layer is set to be 80%-110% of the height of the nanochannel by using a mechanical or chemical method.

In the technical solutions of the present disclosure, the number of the inlets or the outlets is one or more.

A second aspect of the present disclosure provides a method for fabricating the above fluid control device having a micro-nano structure and an electrode structure, which includes the following steps:
1) fabricating the nanochannel array;
   1-i) forming grooves on a first substrate, where a depth of each groove is the height of the nanochannel;
   1-ii) providing an electrode sacrificial layer on a surface of the first substrate, the electrode sacrificial layer having a shape complementary to the intended shape of the electrode;
   1-iii) depositing an electrode layer on the first substrate over the electrode sacrificial layer, and removing the electrode sacrificial layer along with a portion of the electrode structure thereon; and obtaining pairs of electrodes, each pair comprising a first electrode and a second electrode, disposed within or adjacent to the grooves formed in step 1-i) and extending along an extending direction of the nanochannel,
   1-iv) setting a thickness of the electrode to be 80%-110% of the height of the nanochannel.
2) fabricating the microchannel array:
   further forming the microchannel array on the first substrate, wherein the microchannel array comprises the inlet microchannel array and the outlet microchannel array, and the inlet microchannel array and the outlet microchannel array are in fluid communication through the nanochannel array; or
   forming the inlet microchannel array and the outlet microchannel array on a second substrate, wherein the inlet microchannel array includes at least one inlet microchannel, the outlet microchannel array includes at least one outlet microchannel, the inlet is in fluid communication with the inlet microchannel, and the outlet is in fluid communication with the outlet microchannel; aligning and bonding at least one layer of the first substrate comprising the nanochannel array and at least one layer of the second substrate comprising the microchannel array, where the inlet microchannel and the outlet microchannel are in fluid communication through the nanochannel array; providing an inlet and an outlet, wherein the inlet is in fluid communication with the inlet microchannel array, and the outlet is in fluid communication with the outlet microchannel array; alternately stacking and bonding at least one layer of the first substrate comprising the nanochannel array and at least one layer of the second substrate comprising the microchannel array, wherein the inlet microchannel array and the outlet microchannel array are in fluid communication only through the nanochannel array; wherein the outlet and the inlet may be disposed on any layer, provided that the inlet is only in fluid communication with the inlet microchannel array and the outlet is only in fluid communication with the outlet microchannel array; for example, they may be disposed on the second substrate, on the first and second substrates, or on the second and third substrates.

A micro-nano channel network is formed in which the inlet microchannel array and the outlet microchannel array are in fluid communication only through the nanochannel array, such that inflow and outflow occur only via the inlet or the outlet.

In the technical solutions of the present disclosure, connection nodes for the first electrode and the second electrode are further provided in the fluid control device. The first node and the second node are respectively electrically connected to the first electrode array and the second electrode array.

In the technical solutions of the present disclosure, in step 1-iii) or iii), in addition to forming the electrodes, connection lines electrically connected to the first node and the second node are further formed.

In the technical solutions of the present disclosure, the material of the first substrate is a micro-nano fabrication material, preferably glass, quartz, silicon wafer, silicon nitride wafer, silicon dioxide wafer, silicon carbide wafer, polydimethylsiloxane, polymethyl methacrylate, parylene C, polycarbonate, cyclic olefin copolymer, cyclic olefin polymer, polypropylene, photocurable resin, or soft thermoplastic elastomer.

In the technical solutions of the present disclosure, the material of the second substrate is a micro-nano fabrication material, preferably silicon wafer, polydimethylsiloxane, silicon nitride wafer, silicon dioxide wafer, silicon carbide wafer, glass, quartz, polymethyl methacrylate, parylene C, polycarbonate, cyclic olefin copolymer, cyclic olefin polymer, polypropylene, photocurable resin, or soft thermoplastic elastomer.

A third aspect of the present disclosure provides a use of the above fluid control device having a micro-nano structure and an electrode structure for reversible poration of biological particles or loading of an exogenous substance.

A fourth aspect of the present disclosure provides a method for reversible poration and/or loading of biological particles; and

the method includes a step of performing reversible poration on target biological particles by using the above fluid control device having a micro-nano structure and an electrode structure.

In the technical solutions of the present disclosure, the method for loading biological particles further includes a step of mixing an exogenous substance with the target biological particles. Preferably, the mixing step is performed before the biological particles are added to the above fluid control device, or the mixing is performed within the above fluid control device, or the exogenous substance is mixed with the permeabilized biological particles after the biological particles are processed by the above fluid control device.

In the technical solutions of the present disclosure, the loading refers to loading exogenous substance molecules into the biological particles, embedding exogenous substance molecules within a membrane of the biological particles, or attaching exogenous substance molecules to the surface of the membrane of the biological particles.

In the technical solutions of the present disclosure, the biological particles are selected from micro-nano particles having a biological membrane structure, preferably particles having a biological membrane structure with a size of 30 nm-2000 nm, more preferably, extracellular vesicles, membrane vesicles secreted by microorganisms, subcellular particles having membrane structures, cell membrane nanoparticles, artificially synthesized nanoparticles encapsulated by phospholipid bilayer membrane structures, liposomes, or viral vectors.

In the technical solutions of the present disclosure, the exogenous substance is selected from chemotherapeutic agents, biomaterials, nanomaterials, fluorescent dyes, and medical imaging contrast agents.

This application has following beneficial effects.

Firstly, the present disclosure achieves poration of biological particles and loading of exogenous substances based on the synergy of electric field and mechanical stimulation in the micro-nano device. Compared with the existing electroporation-only loading mode, the present disclosure not only utilizes the electroporation mechanism, but also performs poration in combination with mechanical compression. The synergistic multi-physical-field loading mode enables higher loading efficiency of biological particles, which is conducive to satisfying the requirements of animal experiments and clinical applications.

Secondly, in the present disclosure, the height of the nanochannel is designed to be less than, equal to, or slightly greater than the dimensions of biological particles, and the height of the nanochannel is similar to the thickness of the electrodes, such that the biological particles are more likely to form reversible pores under the mechanical compression of the nanochannel and the electric field stimulation of the electrode array. In addition, compared with the high voltage required in conventional electroporation technical solutions, the present disclosure can operate at a lower working voltage. The electrode array within the nanochannel can form a uniform electric field under a lower-voltage condition, thereby effectively avoiding adverse effects on the integrity and biological functionality of the biological particles caused by high voltage or non-uniform electric fields. Meanwhile, avoiding high voltage prevents interference with and damage to exogenous substance molecules, such as degradation or aggregation of nucleic acid molecules. Furthermore, in the prior art, planar electrodes having a thickness far lower than the depth of the microchannel are generally used, which inevitably results in a non-uniform electric field within the channel, leading to different electric field strengths at different spatial positions. Consequently, biological particles are subjected to different electrical stimuli, which affects the degree of membrane pore formation, thereby causing variations in loading efficiency and even damaging the integrity and functionality of the biological particles. In contrast, in the present disclosure, the height of the electrode array is equal to or close to the height of the nanochannel. Compared with two-dimensional planar electrodes, the three-dimensional electrode array can form a uniform electric field environment within the nanochannel, such that biological particles are subjected to substantially the same electric field stimulus, thereby providing more uniform loading performance for the biological particles.

Thirdly, the micro-nano device designed in the present disclosure, by employing the synergistic effect of electrical and mechanical stimulation, can not only satisfy the loading requirements of biological particles having dimensions larger than the characteristic dimensions of the nanochannel, but also enable loading of exogenous substances into extracellular vesicles having smaller dimensions. Due to the fact that the particle size of the biological particles is within a certain range, using mechanical squeezing alone may result in a portion of extracellular vesicles not being subjected to sufficient mechanical forces. The method of the present disclosure can ensure high-efficiency loading.

Fourthly, the mechanical effects in the present disclosure are not limited to mechanical squeezing; through the stacking of micron-scale channels and nano-scale channels, simultaneous effects of mechanical compression, fluid shear, or fluid convection are achieved, thereby applying mechanical forces to biological particles of different dimensions.

Fifthly, the micro-nano device designed in the present disclosure comprises a plurality of microchannels and nanochannels, and each nanochannel includes at least one pair of electrodes therein, which has the advantage of high-throughput processing of biological particle samples such as extracellular vesicles, and is conducive to the promotion of the device in clinical applications.

### BRIEF DESCRIPTION OF THE DRAWINGS

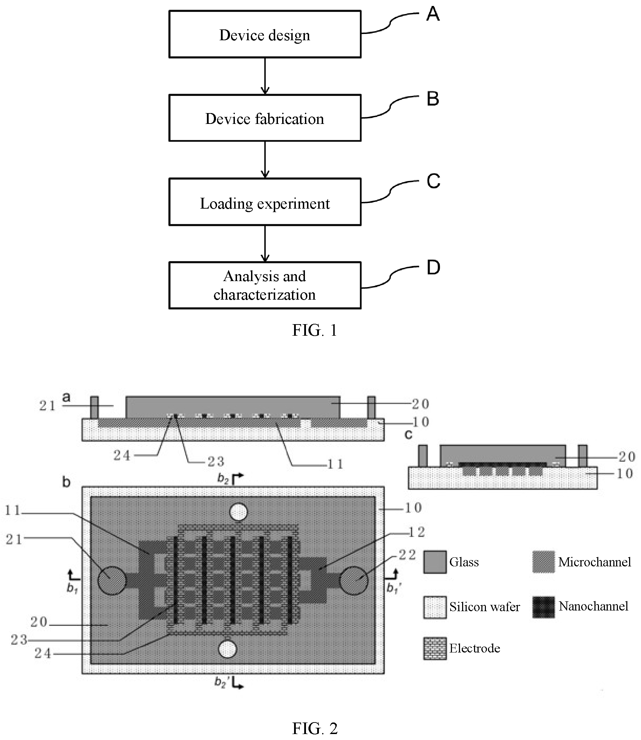

FIG. 1 is an overall flow chart of a technical solution for implementing loading of exogenous substances into biological particles based on a fluid control device having a micro-nano structure and an electrode structure.
FIG. 2 is a structural diagram of a fluid control device having a micro-nano structure and an electrode structure, where a of FIG. 2 is a cross-sectional view taken along line *b₁-b₁'* of view b, b of FIG. 2 is a top view of the fluid control device having a micro-nano structure and an electrode structure, and c of FIG. 2 is a cross-sectional view taken along line *b₂-b₂'* of view b.

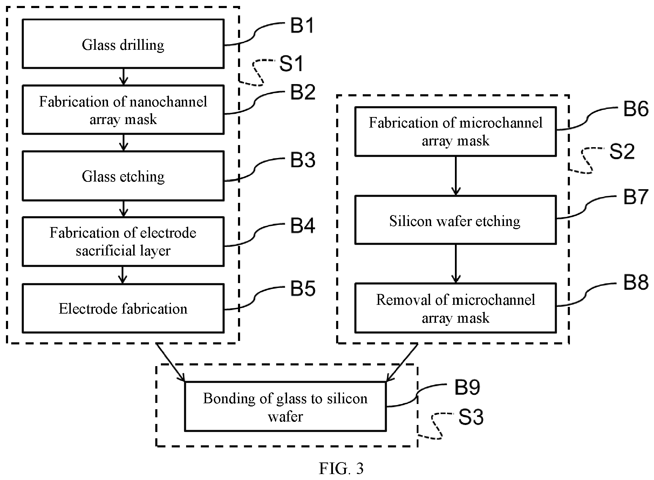

FIG. 3 is a flow chart for fabricating a fluid control device having a micro-nano structure and an electrode structure.

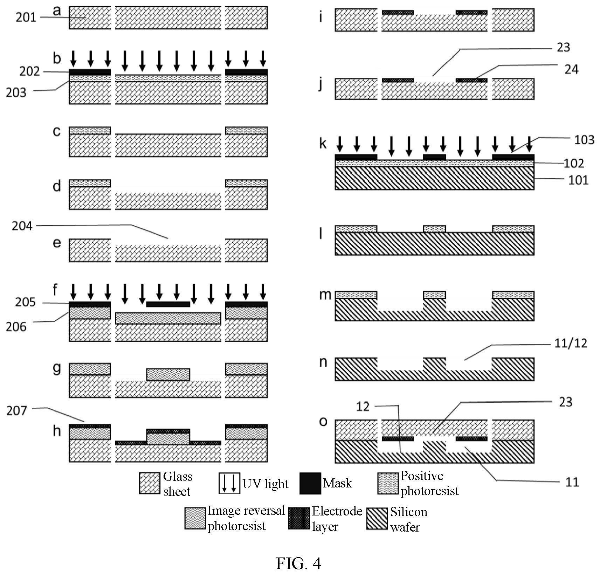

FIG. 4 is a schematic diagram of a fabrication process of a fluid control device having a micro-nano structure and an electrode structure.

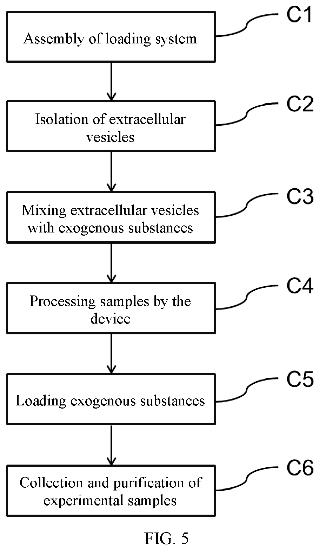

FIG. 5 is a flow chart of a loading experiment using extracellular vesicles as biological particles.

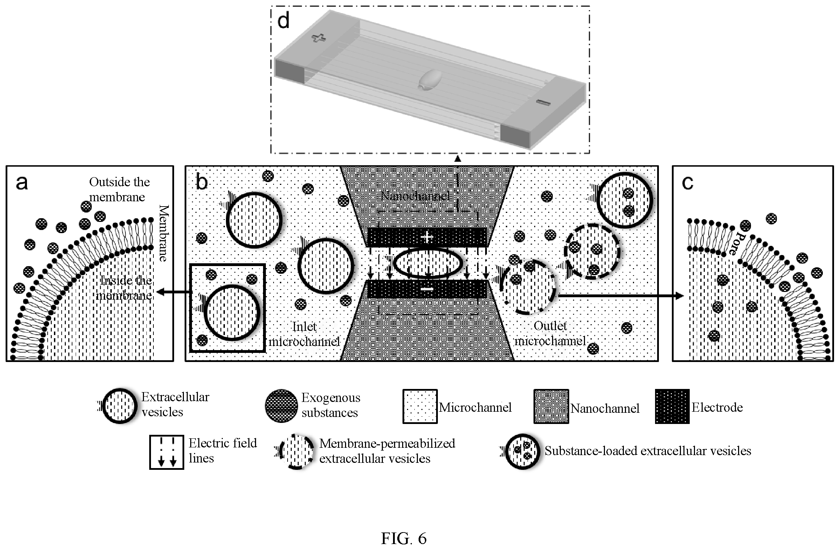

FIG. 6 is a schematic diagram of an experiment in which a fluid control device having a micro-nano structure and an electrode array is used to implement loading of exogenous substances into extracellular vesicles as biological particles, where a of FIG. 6 is an enlarged schematic diagram of the structure of extracellular vesicles before passing through the nanochannel, b of FIG. 6 is a schematic diagram showing mechanical force and electric field acting on extracellular vesicles when passing from the inlet microchannel to the outlet microchannel, c of FIG. 6 is an enlarged schematic diagram of the structure of extracellular vesicles after passing through the nanochannel, and d of FIG. 6 is a 3D diagram of the nanochannel, in which extracellular vesicles are subjected to a substantially uniform electric field under the effects of electrodes on both sides.

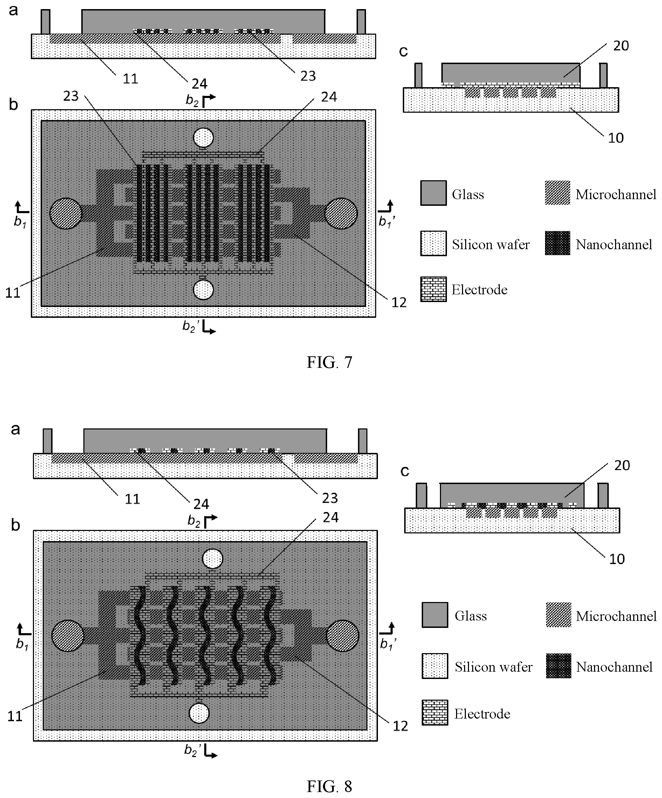

FIG. 7 is an alternative embodiment of a fluid control device having a micro-nano structure and an electrode structure, where a of FIG. 7 is a cross-sectional view taken along line *b₁-b₁'* of view b, b of FIG. 7 is a top view of the fluid control device having a micro-nano structure and an electrode structure, and c of FIG. 7 is a cross-sectional view taken along line *b₂-b₂'* of view b. Each nanochannel includes an array of 4 pairs of electrodes.
FIG. 8 is an alternative embodiment of a fluid control device having a micro-nano structure and an electrode structure, where a of FIG. 8 is a cross-sectional view taken along line *b₁-b₁'* of view b, b of FIG. 8 is a top view of the fluid control device having a micro-nano structure and an electrode structure, and c of FIG. 8 is a cross-sectional view taken along line *b₂-b₂'* of view b. The shape of the nanochannel is serpentine, while the cross-section of the nanochannel is still rectangular.

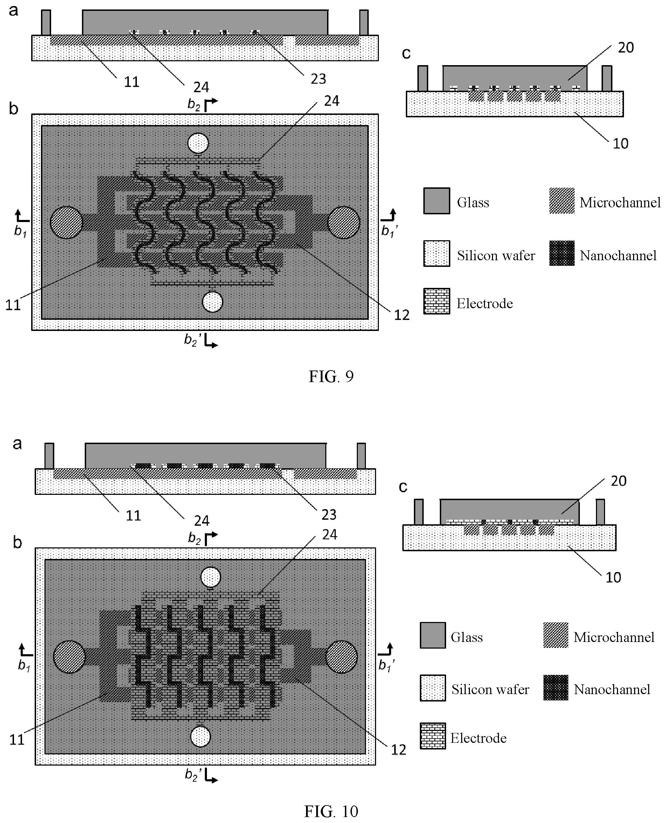

FIG. 9 is an alternative embodiment of a fluid control device having a micro-nano structure and an electrode structure, where a of FIG. 9 is a cross-sectional view taken along line *b₁-b₁'* of view b, b of FIG. 9 is a top view of the fluid control device having a micro-nano structure and an electrode structure, and c of FIG. 9 is a cross-sectional view taken along line *b₂-b₂'* of view b. The shape of the nanochannel is another serpentine shape, while the cross-section of the nanochannel is still rectangular. The electrode arrangement is different from that in the solution of FIG. 8.
FIG. 10 is an alternative embodiment of a fluid control device having a micro-nano structure and an electrode array, where a of FIG. 10 is a cross-sectional view taken along line *b₁-b₁'* of view b, b of FIG. 10 is a top view of the fluid control device having a micro-nano structure and an electrode structure, and c of FIG. 10 is a cross-sectional view taken along line *b₂-b₂'* of view b. The shape of the nanochannel is arcuate, while the cross-section of the nanochannel is still rectangular.

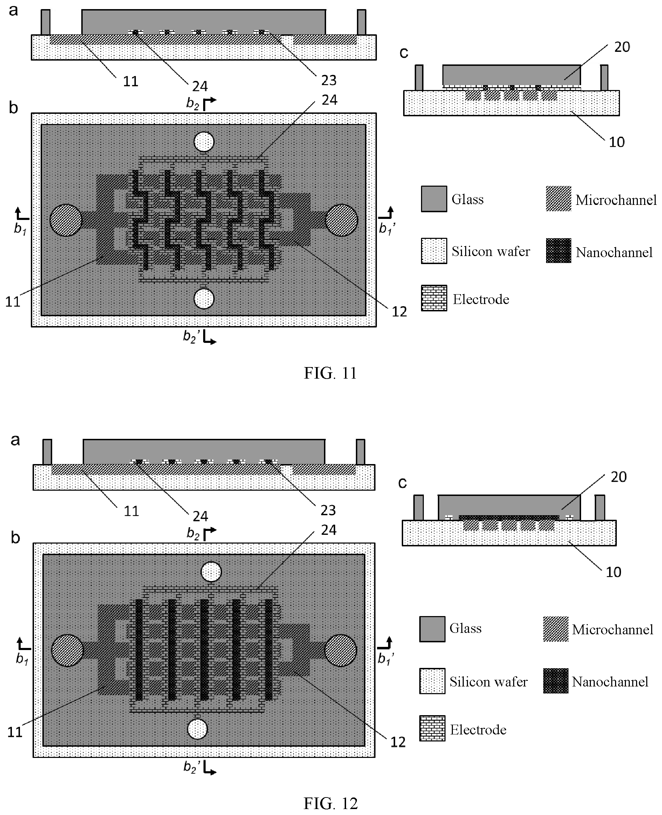

FIG. 11 is an alternative embodiment of a fluid control device having a micro-nano structure and an electrode array, where a of FIG. 11 is a cross-sectional view taken along line *b₁-b₁'* of view b, b of FIG. 11 is a top view of the fluid control device having a micro-nano structure and an electrode structure, and c of FIG. 11 is a cross-sectional view taken along line *b₂-b₂'* of view b. The shape of the nanochannel is arcuate, while the cross-section of the nanochannel is still rectangular. The electrode arrangement is different from that in the solution of FIG. 10.
FIG. 12 is an alternative embodiment of a fluid control device having a micro-nano structure and an electrode structure, where a of FIG. 12 is a cross-sectional view taken along line *b₁-b₁'* of view b, b of FIG. 12 is a top view of the fluid control device having a micro-nano structure and an electrode structure, and c of FIG. 12 is a cross-sectional view taken along line *b₂-b₂'* of view b. The shape of the nanochannel is rectangular, and the cross-section of the nanochannel is trapezoidal.

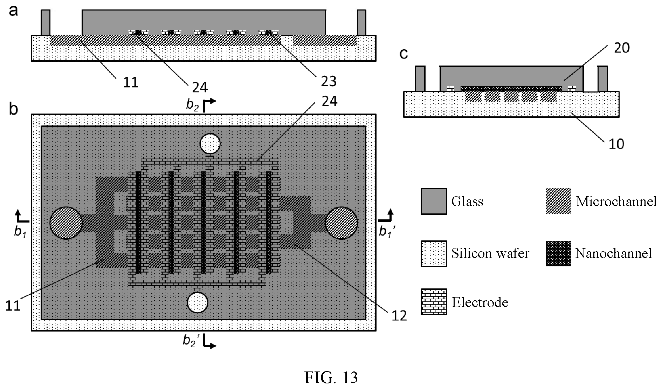

FIG. 13 is an alternative embodiment of a fluid control device having a micro-nano structure and an electrode structure, where a of FIG. 13 is a cross-sectional view taken along line *b₁-b₁'* of view b, b of FIG. 13 is a top view of the fluid control device having a micro-nano structure and an electrode structure, and c of FIG. 13 is a cross-sectional view taken along line *b₂-b₂'* of view b. The shape of the nanochannel is rectangular, and the cross-section of the nanochannel is trapezoidal.

### DETAILED DESCRIPTION OF THE EMBODIMENTS

To make the aforementioned objectives, features, and advantages of the present disclosure more apparent and comprehensible, specific embodiments are described in detail below. However, these embodiments should not be construed as limiting the scope of the present disclosure.

In the present disclosure, the biological particles refer to micro-nano particles having a biological membrane structure with a particle size of 30 nm-2000 nm. For example, the biological particles may be extracellular vesicles, membrane vesicles secreted by microorganisms, subcellular particles having membrane structures, cell membrane nanoparticles, artificially synthesized nanoparticles encapsulated by phospholipid bilayer membrane structures, liposomes, or viral vectors.

The technical solutions of the present disclosure are described below with reference to the drawings. In the present disclosure, a high-throughput micro-nano device based on an electromechanical synergistic effect is designed and fabricated by utilizing micro-nanofluidic technology, with the purpose of achieving high-throughput loading of exogenous substances into target biological particles such as extracellular vesicles, and improving the loading efficiency and loading uniformity thereof.

Some specific embodiments of the present disclosure include four main aspects: device design, device fabrication, loading experiments, and analysis and characterization, as shown in FIG. 1.

First, step A is to perform structural design of the fluid control device having a micro-nano structure and an electrode structure; step B is to fabricate the fluid control device according to the design of step A; step C is to achieve application of the fluid control device through loading experiments, that is, to perform reversible poration of biological membranes of target biological particles and/or loading of exogenous substances into the target biological particles; and step D is to perform analysis and characterization of experimental results.

In some specific embodiments, for the structure of the fluid control device having a micro-nano structure, reference may be made to FIG. 2. Here, a of FIG. 2 is a front cross-sectional view of the fluid control device designed in the present disclosure taken along line *b₁-b₁'* of b of FIG. 2; b of FIG. 2 is a top view of the fluid control device; and c of FIG. 2 is a side cross-sectional view of the fluid control device taken along line *b₂-b₂'* of b of FIG. 2.

The fluid control device having a micro-nano structure and an electrode structure includes an upper substrate layer and a lower substrate layer, and the upper substrate layer and the lower substrate layer are tightly connected. The upper layer is a first substrate 20 provided with a nanochannel array and an electrode array, and the lower layer is a second substrate 10 provided with a microchannel array. The second substrate 10 includes an inlet microchannel array and an outlet microchannel array, the inlet microchannel array includes at least one inlet microchannel 11, and the outlet microchannel array includes at least one outlet microchannel 12. Within the microchannel array layer, the inlet microchannel array and the outlet microchannel array are not in fluid communication with each other. As shown in FIG. 2, the microchannel array layer includes 3 inlet microchannels 11 and 2 outlet microchannels 12.

The first substrate 20 includes a nanochannel array, an electrode array 24, an inlet 21, and an outlet 22. The nanochannel array includes at least one nanochannel 23; a cross-sectional width of the nanochannel ranges from 30 nm to 1000 µm, a cross-sectional height of the nanochannel ranges from 10 nm to 4000 nm, and electrode arrays 24 are respectively disposed adjacent to the nanochannel. The inlet 21 is in fluid communication with the inlet microchannel array, and the outlet 22 is in fluid communication with the outlet microchannel array. The inlet microchannel array and the outlet microchannel array are in fluid communication through the nanochannel array on the first substrate layer.

In some other embodiments, the inlet 21 and the outlet 22, in addition to being disposed in the nanochannel array layer, may also be disposed in the microchannel array layer, as long as the inlet is in fluid communication with the inlet microchannel array and the outlet is in fluid communication with the outlet microchannel array, such that fluid can be delivered to the inlet microchannel array through the inlet and discharged from the outlet microchannel array through the outlet.

In some embodiments, in the fluid control device as shown in FIG. 2, the microchannel array layer is formed by providing a microchannel array on the second substrate. The material of the second substrate is selected from silicon wafer, and may also be selected from other materials capable of achieving micro-nano fabrication, such as polydimethylsiloxane (PDMS), glass, quartz, silicon nitride wafer, silicon dioxide wafer, silicon carbide wafer, polymethyl methacrylate, parylene C, polycarbonate, cyclic olefin copolymer, cyclic olefin polymer, polypropylene, photocurable resin, or soft thermoplastic elastomer.

In some embodiments, the nanochannel array layer of the fluid control device is formed by providing a nanochannel array and an electrode array on the first substrate. The material of the first substrate is selected from glass, and may also be selected from other materials capable of achieving micro-nano fabrication, such as quartz, silicon wafer, silicon nitride wafer, silicon dioxide wafer, silicon carbide wafer, polydimethylsiloxane, polymethyl methacrylate, parylene C, polycarbonate, cyclic olefin copolymer, cyclic olefin polymer, polypropylene, photocurable resin, or soft thermoplastic elastomer.

In the specific embodiment shown in FIG. 2, the nanochannel and the microchannel are arranged at 90° with respect to each other; however, they may also be arranged at other angles, for example, at an angle greater than 0° and less than or equal to 90°.

In the specific embodiment shown in FIG. 2, the circular openings at the left and right ends of the fluid control device are respectively the inlet 21 and the outlet 22, and the circular openings at the upper and lower ends of the device are respectively connection points of positive and negative electrodes. The first substrate (glass layer) includes a plurality of parallel nanochannels, and each nanochannel 23 has a pair of parallel electrodes 24 disposed on both sides thereof, including a first electrode and a second electrode, where the thickness of the electrodes 24 is equal to the height of the nanochannel or is 80%-110% of the height of the nanochannel. The second substrate (silicon wafer) includes a plurality of pairs of microchannels arranged in a parallel interdigitated configuration, where the inlet 21 on the left side is fluid communication with a plurality of inlet microchannels 11, and the outlet 22 on the right side is fluid communication with a plurality of outlet microchannels 12; the inlet microchannels and the outlet microchannels are independent of and not in fluid communication with each other. After the bottom of the glass is bonded to the top of the silicon wafer, the nanochannel array can bring the inlet microchannels and the outlet microchannels into fluid communication.

In the specific embodiment shown in FIG. 2, the microchannels are arranged in a parallel interdigitated configuration, and the nanochannels are arranged perpendicular to the microchannels, with the nanochannels arranged in parallel with each other. A plurality of nanochannels that connect the inlet microchannel and the outlet microchannel are provided between any inlet microchannel and outlet microchannel.

In some other embodiments of the present disclosure, the number of the inlet and the outlet is not limited to one, and may be plural, so as to allow target biological particles and exogenous substances to enter through a plurality of inlets and flow out through a plurality of outlets.

In some other embodiments of the present disclosure, the shape of the microchannel array is not limited to the parallel interdigitated configuration, and may also be a vertical array, a circumferential array, a spiral array, a curved array, or a non-parallel linear array.

In some other embodiments of the present disclosure, the shape of the nanochannel array is not limited to the parallel arrangement, and any geometric relationship among the nanochannels is acceptable as long as the objective of fluidically connecting the inlet microchannel and the outlet microchannel is achieved.

In some other embodiments of the present disclosure, the shape of the electrode array is not limited to the parallel arrangement, as long as at least one pair of electrodes can be disposed adjacent to or within each nanochannel.

In some specific embodiments of the present disclosure, the nanochannel has a relatively large width, and the first electrodes and the second electrodes are arranged in an interdigitated and staggered manner along a fluid flow direction within the nanochannel; among the first electrodes and the second electrodes, at least one type of electrode has a number greater than or equal to 1, and a passage allowing target biological particles to pass therethrough is formed between the staggered first electrodes and second electrodes. Specifically, as shown in FIG. 7, the nanochannel is provided with a plurality of pairs of electrodes, where the first electrode array electrically connected to the upper first node includes 2 first electrodes, and the second array electrically connected to the lower second node includes 3 second electrodes. The first electrodes and the second electrodes of the above 5 electrodes are arranged in a staggered manner to form 4 pairs of electrodes, and a certain width is present between the first electrode and the second electrode to form a passage through which target biological particles can pass.

In some specific embodiments of the present disclosure, the first electrode and the second electrode are respectively disposed adjacent to the nanochannel and arranged along the shape of the nanochannel. For example, in FIGS. 7-11, as the nanochannel bends, the electrodes are also configured to be curved. The electrodes may have identical cross-sectional shapes at every cross-section, all of which are rectangular, for example, as shown in FIGS. 2, 7-9, and 11; alternatively, they may have identical cross-sectional shapes that are not rectangular, for example, as shown in FIGS. 12-13; or they may have different cross-sectional shapes or sizes at different cross-sections, for example, as shown in FIG. 10, as long as the electrodes can provide a substantially uniform electric field when target biological particles pass through the nanochannel.

In some specific embodiments of the present disclosure, the nanochannel may be linear or curved, for example, serpentine as shown in FIG. 8 or FIG. 9, or arcuate as shown in FIG. 10 and FIG. 11. Alternatively, other shapes as disclosed in Chinese Patent CN 110975953 B may be adopted, for example, radial shapes as shown in figure 5 or figure 6 thereof. The nanochannel may take any form as long as it can fluidically connect the inlet microchannel array and the outlet microchannel array.

In some specific embodiments of the present disclosure, the cross-sectional shape of the nanochannel may be rectangular, for example, as shown in FIGS. 2 and 7-10, or may be square or trapezoidal, for example, as shown in FIG. 12 and 13, or may be triangular, circular, elliptical, or the like.

In some other embodiments of the present disclosure, the microchannel array and the nanochannel array are disposed in the same layer, and the inlet microchannel array and the outlet microchannel array are in fluid communication only through the nanochannel array.

In some other embodiments of the present disclosure, the numbers of the microchannels and the nanochannels are not limited and may be further expanded to different quantities according to high-throughput requirements; and the geometric relationship between the nanochannel array and the microchannel array in the horizontal direction is not limited to perpendicularity, as long as the nanochannels intersect and fluidically connect the microchannels.

In some embodiments of the present disclosure, the nanochannel in the nanochannel array is dimensioned such that its width is equal to or greater than the particle size of the target biological particles, and its height is less than, equal to, or greater than the particle size of the target biological particles.

In some embodiments of the present disclosure, the width of the nanochannel ranges from 1 to 10,000 times the particle size of the target biological particles, preferably 200-1000 times, e.g., 20 times, 30 times, 50 times, 100 times, 200 times, 500 times, 600 times, 700 times, 800 times, 900 times, or 1000 times.

In some embodiments of the present disclosure, the width of the nanochannel ranges from 30 nm to 1000 µm, preferably ranging from 2 µm to 100 µm, e.g., 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 25 µm, 30 µm, 40 µm, 50 µm, 60 µm, 70 µm, 80 µm, 90 µm, or 100 µm.

In some embodiments of the present disclosure, the height of the nanochannel is less than the particle size of the target biological particles.

In some embodiments of the present disclosure, the height of the nanochannel is less than the particle size of the target biological particles and greater than or equal to 0.2 times said particle size, preferably greater than or equal to 0.5 times, 0.6 times, 0.7 times, 0.8 times, or 0.9 times the particle size of the target biological particles.

In some embodiments of the present disclosure, the particle size of the target biological particles is an average particle size, preferably an arithmetic mean diameter or a median diameter.

In some embodiments of the present disclosure, the height of the nanochannel ranges from 10 nm to 4000 nm, e.g., 10 nm, 20 nm, 30 nm, 40 nm, 50 nm, 60 nm, 70 nm, 80 nm, 90 nm, 100 nm, 200 nm, 300 nm, 400 nm, 500 nm, 600 nm, 700 nm, 800 nm, 900 nm, 1000 nm, 1200 nm, 1400 nm, 1600 nm, 1800 nm, 2000 nm, 2500 nm, 3000 nm, 3500 nm, or 4000 nm.

In some embodiments of the present disclosure, the height of the nanochannel is less than the width of the nanochannel.

FIG. 3 illustrates a fabrication process of a fluid control device having a micro-nano structure and an electrode structure by using micro-nanofluidic technology, which includes three steps:
S1, fabricating a first substrate layer;
S2, fabricating a second substrate layer; and
S3, bonding the first substrate layer and the second substrate layer to fabricate the fluid control device.

Step S1 is to fabricate a first substrate comprising a nanochannel array, an outlet, an inlet, and electrodes, so as to obtain a nanochannel array layer, where glass is used as the first substrate. Step S2 is to fabricate a second substrate comprising a microchannel array, so as to obtain a microchannel array layer, where a silicon wafer is used as the second substrate. Step S3 is bonding the nanochannel array layer and the microchannel array layer obtained in steps S1 and S2.

The order of steps S1 and S2 may be changed. For example, the first substrate layer may be fabricated first, the second substrate layer may be fabricated first, or the two substrate layers may be fabricated simultaneously.

As shown in FIG. 3, the main fabrication steps of step S1 include: B1 glass drilling, B2 fabrication of nanochannel array mask, B3 glass etching, B4 fabrication of electrode sacrificial layer, and B5 electrode fabrication, as shown in S1 of FIG. 3. The fabrication steps of step S2 include: B6 fabrication of microchannel array mask, B7 silicon wafer etching, and B8 removal of microchannel array mask, as shown in S2 of FIG. 3. Step S3 includes B9 cleaning the fabricated glass and silicon wafer, and then aligning the bottom of the glass layer with the top of the silicon wafer layer and bonding the same, thereby completing fabrication of the fluid control device, as shown in S3 of FIG. 3.

A schematic structural diagram of the specific fabrication steps is shown in FIG. 4, where in step S1, a first substrate 201 (e.g., glass layer) comprising a nanochannel array is fabricated, i.e., fabrication of the nanochannel array layer, and this step includes sub-steps B1-B5.

In sub-step B1, glass drilling is performed.

Firstly, specific positions of a glass sheet are drilled by using a laser or an etching method to form an inlet and an outlet of the device, as well as a first node and a second node for connecting the electrode array, as shown in a of FIG. 4.

In sub-step B2, a nanochannel array mask is fabricated.

The drilled glass sheet is cleaned and dried, then the surface of the glass sheet is subjected to hydrophilic treatment and spin-coated with an adhesion promoter layer. After that, the surface thereof is spin-coated with a layer of positive photoresist 203 and baked, and finally exposure treatment is performed by using a first mask 202, as shown in b of FIG. 4. The exposed glass sheet is immersed in a developer solution for development, and hard baking is performed at a certain temperature for a period of time to harden the photoresist, thereby obtaining a nanochannel array mask, as shown in c of FIG. 4.

In sub-step B3, glass etching is performed.

The glass sheet including the nanochannel array mask is etched, thereby etching a groove array 204 on the surface of the glass sheet, as shown in d of FIG. 4.

In sub-step B4, an electrode sacrificial layer is fabricated.

The etched glass sheet is cleaned to remove the nanochannel array mask on the surface, as shown in e of FIG. 4; after drying, the glass sheet is further subjected to hydrophilic treatment and spin-coated with an adhesion promoter layer. After that, the surface thereof is spin-coated with a layer of image reversal photoresist 206, followed by pre-baking, exposure using a second mask 205, reversal baking, and flood exposure, as shown in f of FIG. 4. The glass sheet after flood exposure is immersed in a developer solution for development, then cleaned with deionized water and blow-dried, and finally a photoresist bottom film is removed by using an etching method, thereby obtaining an electrode sacrificial layer, as shown in g of FIG. 4.

In sub-step B5, electrodes are fabricated.

After the electrode sacrificial layer is fabricated, a relatively thin metal layer is first deposited on the surface of the glass sheet as an adhesion layer, where the adhesion layer generally employs chromium or titanium, and then a relatively thick metal layer is deposited on the surface of the adhesion layer as an electrode layer 207, the electrode layer generally being a metal having good conductivity, such as gold, platinum, or copper, as shown in h of FIG. 4. The glass sheet on which the metal thin film is deposited is immersed in an organic solvent or a stripping solution, and then subjected to low-power ultrasonication until the metal layer is completely stripped off, as shown in i of FIG. 4. If the thickness of the electrode is different from the thickness of the nanochannel, that is, the thickness of the electrode is lower than the surface of the first substrate glass sheet, the glass sheet with the deposited electrode layer is subjected to chemical mechanical planarization treatment, such that the thickness of the electrode is the same as the height of the nanochannel, thus completing fabrication of the electrodes 24. The two electrodes and the first substrate together form a nanochannel 23, as shown in j of FIG. 4, thereby obtaining the nanochannel array layer.

During the fabrication process of the nanochannel, to further improve the experimental processing throughput, the height of the nanochannel is controlled to be less than, equal to, or greater than the particle size of the target biological particles, while the width of the nanochannel is controlled to be equal to or greater than the particle size of the target biological particles, thereby achieving fabrication of the nanochannel by using conventional processes without using high-precision instruments and equipment, such as ultra-high-precision lithography machines. Since the fluid control device according to the present disclosure is a micro-nano device having small characteristic dimensions, the fabrication process thereof cannot be simply analogized to devices having conventional dimensions. In the prior art, micron-scale fluid channels are generally employed precisely due to limitations of the fabrication process. The present disclosure overcomes this technical problem by controlling the nano-scale height of the nanochannels, thereby achieving application of mechanical stimulation to the target biological particles and simultaneously achieving effective control of uniform electric field distribution. In the prior art, the depth of microfluidic channels is relatively large, but there is a severe dimensional mismatch problem between the thickness of the electrodes and the depth of the microfluidic channels, resulting in non-uniform electric field stimulation on biological particles when passing through the electric field. Therefore, the present disclosure employs electrodes having the same height as the nanochannel to ensure that a more uniform electric field is generated thereby; meanwhile, by controlling the width of the nanochannel to be equal to or greater than the particle size of the target biological particles, the nanochannels are ensured to have characteristics of high throughput. In addition, by arranging a plurality of nanochannels in fluid communication with the microchannels, a plurality of nanochannels are established between the inlet microchannel and the outlet microchannel, thereby further improving throughput.

In step S2, a second substrate layer 101 (e.g., silicon wafer) comprising a microchannel array is fabricated, i.e., fabrication of the microchannel array layer, and this step includes sub-steps B6-B8.

In sub-step B6, a microchannel array mask is fabricated.

First, the second substrate 101 (e.g., silicon wafer) is cleaned and dried, then the surface of the silicon wafer is subjected to hydrophilic treatment, and then spin-coated with a layer of positive photoresist 102 and baked. Finally, exposure treatment is performed by using a third mask 103, as shown in k of FIG. 4. The exposed substrate is directly immersed in a developer solution for development, and then hard baking is performed at a certain temperature for a period of time to harden the photoresist, thereby obtaining a microchannel array mask, as shown in l of FIG. 4.

In sub-step B7, silicon wafer etching is performed.

The silicon wafer coated with the microchannel array mask is etched, thereby etching a microchannel array on the surface of the silicon wafer, as shown in m of FIG. 4.

In sub-step B8, the microchannel array mask is removed.

The etched silicon wafer is immersed in an organic solvent or a photoresist stripping solution to remove the microchannel array mask on the surface of the silicon wafer. After drying, a silicon wafer layer comprising a microchannel array 11 or 12 is obtained, as shown in n of FIG. 4, thereby obtaining the microchannel array layer.

In step S3, chip bonding is performed, and this step includes sub-step B9.

In sub-step B9, the glass layer is bonded to the silicon wafer layer.

First, the glass sheet and the silicon wafer are cleaned, then the bottom of the glass sheet and the top of the silicon wafer are attached and aligned, and then bonding is performed by using a bonding machine. Finally, the fluid control device having a micro-nano structure and an electrode structure can be obtained, as shown in o of FIG. 4.

The loading method is shown in step C of FIG. 5, and loading experiments are described as follows.

After fabrication of the above fluid control device is completed, the same can be used for loading of exogenous substances into biological particles. FIG. 5 illustrates a specific loading method by taking extracellular vesicles as an example. The loading method includes: C1 assembly of loading system, C2 isolation of extracellular vesicles, C3 mixing extracellular vesicles with exogenous substances, C4 processing samples by the device, C5 loading exogenous substances, and C6 collection and purification of experimental samples.

In sub-step C1, a loading system is assembled.

The fluid control device is fabricated by using the above steps in the micro-nanofluidic technology. First, the fluid control device is required to be assembled with a metal fixture, then the inlet and the outlet of the device are respectively connected to tubes, and finally electrode connection points are respectively connected to the positive electrode and the negative electrode of a direct-current power supply through wires, thereby completing the assembly.

In sub-step C2, extracellular vesicles are isolated.

Before loading exogenous substances into extracellular vesicles, extracellular vesicles also need to be separated and isolated from biological samples. Commonly used biological samples include cell culture supernatants, plasma, serum, urine, saliva, cerebrospinal fluid, ascites, amniotic fluid, semen, synovial fluid, bronchial fluid, tears, bile, gastric acid, lymph, pleural effusion, gastrointestinal lavage fluid, bronchoalveolar lavage fluid, milk, grape, grapefruit, lemon, watermelon, carrot, ginger, tomato, broccoli, ginseng, and the like. The isolation methods include commonly used extracellular vesicle separation methods such as ultracentrifugation, density gradient centrifugation, filtration, immunocapture, precipitation kits, size exclusion chromatography separation methods, micro-nanofluidic separation methods, and polymer precipitation separation. Finally, the extracellular vesicles are resuspended in a biological solution such as phosphate-buffered saline, cell culture medium, or normal saline for later use.

In some other embodiments, the extracellular vesicles may also be replaced with other biological particles, such as micro-nano particles having a biological membrane structure with a particle size of 30 nm-2000 nm. For example, the biological particles may be membrane vesicles secreted by microorganisms, subcellular particles having membrane structures, cell membrane nanoparticles, artificially synthesized nanoparticles encapsulated by phospholipid bilayer membrane structures, liposomes, or viral vectors.

In some other embodiments, the extracellular vesicles may be replaced with other biological particles, and the biological particles may also be commercial products or samples obtained by pre-preparation.

In sub-step C3, the extracellular vesicles are mixed with exogenous substances.

An extracellular vesicle solution at a certain concentration is mixed with an exogenous substance solution at a certain concentration, and then the uniformly mixed solution is injected through a tube into the inlet microchannel of the high-throughput micro-nano device. Due to the blocking effect of the extracellular vesicle membrane, the exogenous substance molecules can only be distributed outside the extracellular vesicles, as shown in a of FIG. 6.

In the present disclosure, the exogenous substances generally refer to exogenous substance molecules having a dimension less than or equal to 500 nm, selected from chemotherapeutic agents, biomaterials, fluorescent dyes, nanomaterials, medical imaging contrast agents, and the like. Since reversible pores are formed on the membrane of target biological particles to allow the exogenous substances to enter the target biological particles according to the present disclosure, any active ingredient having a particle size less than 500 nm can enter the biological particles through the temporary pores on the membrane thereof.

The chemotherapeutic agents are selected from small-molecule compounds for treating different diseases, such as antitumor compounds, infectious disease drugs, cardiovascular and cerebrovascular disease therapeutic agents, neurodegenerative disease therapeutic agents, autoimmune disease therapeutic agents, antimicrobial therapeutic agents, antiviral therapeutic agents, and blood glucose-regulating compounds.

Examples of antitumor compounds include, but are not limited to, alkylating agents (e.g., cyclophosphamide), doxorubicin, curcumin, and paclitaxel; alkyl sulfonates, e.g., busulfan, improsulfan, and piposulfan; aziridines, e.g., benzodopa, carboquone, meturedopa, and uredopa; ethyleneimines and methylmelamines, including altretamine, triethylenemelamine, triethylenephosphoramide, triethylenethiophosphoramide, and trimethylolomelamine; acetogenins (especially bullatacin and bullatacinone); camptothecin and analogs thereof, e.g., topotecan; bryostatin; callystatin; CC-1065 (including synthetic analogs thereof, adozelesin, carzelesin, and bizelesin); cryptophycin (particularly cryptophycin 1 and cryptophycin 8); dolastatin; duocarmycin (including synthetic analogs KW-2189 and CB1-TM1); eleutherobin; pancratistatin; sarcodictyin; spongistatin; nitrogen mustards, e.g., chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, and uracil mustard; nitrosoureas, e.g., carmustine, chlorozotocin, fotemustine, lomustine, nimustine, and ranimustine; antibiotics, e.g., enediyne antibiotics (e.g., calicheamicin, particularly calicheamicin γlI and calicheamicin ωl1); dynemicin, including dynemicin A; bisphosphonates, e.g., clodronate; esperamicin; neocarzinostatin chromophore and related chromoprotein enediyne antibiotic chromophores, aclacinomycin, actinomycin, anthramycin, azaserine, bleomycin, cactinomycin, carabicin, carminomycin, carzinophilin, chromomycin, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin, and deoxydoxorubicin), epirubicin, esorubicin, idarubicin, marcellomycin, mitomycin (e.g., mitomycin C), mycophenolic acid, nogalamycin, olivomycin, peplomycin, porfiromycin, puromycin, quelamycin, rodorubicin, streptonigrin, streptozocin, tubercidin, ubenimex, zinostatin, and zorubicin; antimetabolites, e.g., methotrexate and 5-fluorouracil (5-FU); folate analogs, e.g., denopterin, methotrexate, pteropterin, and trimetrexate; purine analogs, e.g., fludarabine, 6-mercaptopurine, thiamiprine, and thioguanine; pyrimidine analogs, e.g., ancitabine, azacitidine, 6-azauridine, carmofur, cytarabine, dideoxyuridine, doxifluridine, enocitabine, and floxuridine; androgens, e.g., calusterone, dromostanolone propionate, epitiostanol, mepitiostane, and testolactone; anti-adrenal agents, e.g., aminoglutethimide, mitotane, and trilostane; folic acid supplements, e.g., leucovorin; aceglatone; aldophosphamide glycoside; aminolevulinic acid; eniluracil; amsacrine; bestrabucil; bisantrene; edatrexate; defofamide; demecolcine; diaziquone; eflornithine; elliptinium acetate; epothilone; etoglucid; gallium nitrate; hydroxycarbamide; lentinan; lonidamine; maytansinoids, e.g., maytansine and ansamitocin; mitoguazone; mitoxantrone; mopidamol; nitracrine; pentostatin; phenamet; pirarubicin; losoxantrone; podophyllinic acid; 2-ethylhydrazide; procarbazine; PSK polysaccharide complex; razoxane; rhizoxin; sizofuran; spirogermanium; tenuazonic acid; triaziquone; 2,2',2"-trichlorotriethylamine; trichothecene (particularly T-2 toxin, verrucarin, roridin, and anguidine); urethan; vindesine; dacarbazine; mannomustine; mitobronitol; mitolactol; pipobroman; ganciclovir; arabinoside ("Ara-C"); cyclophosphamide; thiotepa; taxoid, e.g., paclitaxel and docetaxel; chlorambucil; gemcitabine; 6-thioguanine; mercaptopurine; methotrexate; platinum coordination complexes, e.g., cisplatin, oxaliplatin, and carboplatin; vinblastine; platinum; etoposide (VP-16); ifosfamide; mitoxantrone; vincristine; vinorelbine; novantrone; teniposide; edatrexate; daunomycin; aminopterin; Xeloda; ibandronate; irinotecan (e.g., CPT-11); topoisomerase inhibitor RFS 2000; difluoromethylornithine (DFMO); retinoids, e.g., retinoic acid; capecitabine; and pharmaceutically acceptable salts, acids, or derivatives of any one of the foregoing.

Examples of drugs for treating infectious diseases include, but are not limited to, amphotericin B, ciprofloxacin, rifampicin, tobramycin, and the like.

Examples of drugs for treating cardiovascular diseases include, but are not limited to, amiodarone, atenolol, isosorbide-5-mononitrate, and the like.

Examples of drugs for treating neurodegenerative diseases include, but are not limited to, tanshinone IIA, levodopa, donepezil, memantine, and the like.

Examples of drugs for treating autoimmune diseases include, but are not limited to, tacrolimus, dexamethasone sodium phosphate, and the like.

Examples of antimicrobial therapeutic agents include, but are not limited to, β-lactams, aminoglycosides, macrolides, tetracyclines, fluoroquinolones, lincosamides, glycylcyclines, chloramphenicol-like compounds, and the like.

Examples of antiviral therapeutic agents include, but are not limited to, oseltamivir, baloxavir marboxil, zanamivir, peramivir, and the like.

Examples of blood glucose-regulating compounds include, but are not limited to, insulin and analogs thereof, sulfonylurea secretagogues, metformin compounds, α-glucosidase inhibitors, thiazolidinedione derivative sensitizers, anisic acid derivative secretagogues, GLP-1 receptor agonists, DPP-4 enzyme inhibitors, and the like.

The biomaterials are selected from cytokines, proteins, polypeptides, enzymes, nucleic acids, probes, and the like, for example, protein drug models such as immunoglobulins, interleukins, bovine serum albumin, endonucleases, and Cas9 proteins; nucleic acid drug models such as plasmids, ribonucleic acid, deoxyribonucleic acid, and oligonucleotides; and biomolecules such as potassium ion probe molecules, calcium ion probe molecules, and inositol triphosphate.

The nanomaterials are selected from nanomaterial drug models such as nano metallic particles, quantum dots, carbon nanotubes, carbon dots, phosphorus dots, molybdenum disulfide, MXene, magnetic nanoparticles, and nano fluorescent probes.

The medical imaging contrast agents are selected from near-infrared fluorescent contrast agents (for example, indocyanine green and heptamethine cyanine small-molecule IR-780), X-ray computed tomography contrast agents (for example, iohexol, iopromide, iothalamate, and iodixanol), magnetic resonance imaging contrast agents (for example, paramagnetic contrast agents and superparamagnetic contrast agents), radionuclide contrast agents, and other carrier-encapsulated contrast agents.

In sub-step C4, the sample is processed by using the device.

When the mixed solution reaches the nanochannel through the inlet microchannel, the extracellular vesicles are subjected to mechanical constriction of the nanochannel and electric field generated by adjacent electrode arrays while passing therethrough. As a result, phospholipid molecules of the extracellular vesicles are rearranged under the synergistic effect of the mechanical squeezing and the electric field stimulation to form temporary pores, which provide pathways for loading exogenous substances, as shown in b of FIG. 6. FIG. 6 is merely a schematic diagram, and the dimensions and proportions thereof are set according to actual requirements, where the lower diagram (b) shows a top view, and the upper diagram (d) shows a side view. The depth of the nanochannel is set to be less than, equal to, or greater than the particle size of the target biological particles, while the width of the nanochannel is set to be greater than or equal to the particle size of the target biological particles. When the sample solution continuously flows into the inlet microchannel, the biological particles therein pass through the nanochannel along with the fluid, and the biological particles are squeezed and deformed in the height direction due to the mechanical constriction of the nanochannel. The width of the nanochannel is the same as or greater than the particle size of the target biological particles, such that efficient passage of a large number of biological particles is ensured, and high-throughput sample preparation is achieved. Furthermore, since the height of the nanochannel is far lower than the channel depth of microfluidic chips in the prior art, positions of the biological particles within the electric field and the spatial extent of the electric field of the electrodes are effectively controlled, thereby ensuring that the biological particles are subjected to more uniform electric field stimulation when passing through the nanochannel.

In sub-step C5, exogenous substances are loaded.

When the extracellular vesicles move from the nanochannel to the outlet microchannel, a large number of temporary pores are formed on the membrane surface of the extracellular vesicles. Exogenous substances enter the interior of the extracellular vesicles from the exterior through the pores under the action of a concentration gradient and fluid convection. When the extracellular vesicles in the outlet microchannel are no longer subjected to the electromechanical synergistic effect, phospholipid molecules on the surface of the extracellular vesicles are rearranged again. Consequently, the pores on the membrane surface gradually disappear and an intact form is restored, thereby achieving the loading of exogenous substances into the extracellular vesicles, as shown in c of FIG. 6.

In sub-step C6, experimental samples are collected and purified.

The processed mixed solution is collected, and then extracellular vesicles in the mixed solution are isolated and purified by methods such as ultracentrifugation, density gradient centrifugation, filtration, immunocapture, precipitation kits, size exclusion chromatography separation methods, micro-nanofluidic separation methods, and polymer precipitation separation, thereby obtaining the loaded extracellular vesicles.

In step D, analysis and characterization are performed.

After extracellular vesicles are obtained through processing by the high-throughput micro-nano device, the morphology of the extracellular vesicles, the loading efficiency of the extracellular vesicles, the mass or quantity of loaded exogenous substances, the zeta potential of the extracellular vesicles, and the like can be characterized by analysis methods such as a nano flow cytometer, a flow cytometer, a super-resolution optical imaging system, a transmission electron microscope, an atomic force microscope, a cryo-electron microscope, a nanoparticle tracking analyzer, a fluorescent dye kit, a real-time fluorescent quantitative polymerase chain reaction instrument, a mass spectrometer, and a spectrophotometer. In addition, the integrity and biological activity of the loaded extracellular vesicles can also be investigated through in vitro cell assays and in vivo animal experiments, so as to verify the application potential of the high-throughput micro-nano device in fields such as biomedical research and clinical treatment.

## Claims

1. A fluid control device having a micro-nano structure and an electrode structure, the fluid control device comprising a microchannel array, a nanochannel array, and an electrode array, wherein
the microchannel array comprises an inlet microchannel array and an outlet microchannel array; the inlet microchannel array comprises at least one inlet microchannel, and the outlet microchannel array comprises at least one outlet microchannel; and the nanochannel array comprises at least one nanochannel;
the inlet microchannel array and the outlet microchannel array are in fluid communication only through the nanochannel array to form a network, the micro-nano channel network permitting fluid inflow or outflow occurs only via an inlet or an outlet;
the inlet microchannel array is in fluid communication with the inlet, and the outlet microchannel array is in fluid communication with the outlet;
the electrode array comprises a first electrode array and a second electrode array, the first electrode array comprises at least one first electrode, the second electrode array comprises at least one second electrode, and the first electrode and the second electrode are arranged in pairs and configured to form a substantially uniform electric field between the first electrode and the second electrode;
the first electrode array is electrically connected to a first node, and the second electrode array is electrically connected to a second node; the first node and the second node are each electrically connected to a power supply; and
the nanochannel is dimensioned such that a width of the nanochannel is greater than or equal to a particle size of target biological particles; a height of the nanochannel is 20%-200% of the particle size of the target biological particles; the first electrode and the second electrode are disposed within or adjacent to the nanochannel; a thickness of the first electrode and a thickness of the second electrode are 80%-110% of the height of the nanochannel; the first electrode and the second electrode are configured to provide a substantially uniform electric field for the target biological particles passing through the nanochannel;
preferably, the electrode array is disposed within the nanochannel, and each nanochannel comprises at least one pair of the first electrode and the second electrode; and the first electrode and the second electrode are disposed adjacent to and/or within the nanochannel along a fluid flow direction within the nanochannel;
preferably, the first electrode array and the second electrode array respectively comprise a plurality of first electrodes and a plurality of second electrodes, and the first electrode array and the second electrode array are arranged in an interdigitated parallel configuration.

2. The fluid control device according to claim 1, wherein the microchannel is dimensioned such that a depth and a width of the microchannel are both greater than the particle size of the target biological particles, and the depth and the width of the microchannel are same or different;
preferably, the microchannel array and the nanochannel array are respectively disposed on different substrate layers, or are disposed on a same substrate layer;
preferably, the microchannel array and the nanochannel array are respectively disposed on different substrate layers or on a same substrate layer, and the microchannel array and the nanochannel array are alternately stacked;
preferably, the microchannel array and the nanochannel array are respectively disposed on different substrate layers or on a same substrate layer, and the substrate comprising the microchannel array comprises at least one layer.

3. The fluid control device according to any one of claims 1 and 2, wherein a length of the electrode is greater than or equal to a length of the nanochannel;
preferably, the nanochannel is dimensioned such that the width of the nanochannel ranges from 1 to 10,000 times a particle size of target biological particles.

4. The fluid control device according to any one of claims 1 to 3, wherein a width of the first electrode or the second electrode of the electrode array is greater than or equal to 1 µm; preferably 1 µm-100 µm;
preferably, when the first electrode and the second electrode are disposed adjacent to the nanochannel, the first electrode, the second electrode, and a substrate together constitute the nanochannel, and a distance between the first electrode and the second electrode is equal to the width of the nanochannel, preferably 30 nm-1000 µm;
preferably, when at least one of the first electrode and the second electrode is disposed in a middle of the nanochannel, a distance between an adjacent first electrode and second electrode ranges from 30 nm to 1000 µm.

5. The fluid control device according to any one of claims 1 to 4, wherein a material for fabricating the nanochannel array is a micro-nano fabrication material, and a material for fabricating a layer of the microchannel array is a micro-nano fabrication material;
preferably, the material for fabricating the nanochannel array is selected from the group consisting of glass, quartz, silicon wafer, silicon nitride wafer, silicon dioxide wafer, silicon carbide wafer, polydimethylsiloxane, polymethyl methacrylate, parylene C, polycarbonate, cyclic olefin copolymer, cyclic olefin polymer, polypropylene, photocurable resin, and soft thermoplastic elastomer; and
preferably, the material for fabricating a layer of the microchannel array is selected from the group consisting of silicon wafer, polydimethylsiloxane, silicon nitride wafer, silicon dioxide wafer, silicon carbide wafer, glass, quartz, polymethyl methacrylate, parylene C, polycarbonate, cyclic olefin copolymer, cyclic olefin polymer, polypropylene, photocurable resin, and soft thermoplastic elastomer.

6. The fluid control device according to any one of claims 1 to 5, wherein the electrode is deposited on a substrate by a thin film growth method; and preferably, the thin film growth method comprises sputtering, evaporation, chemical vapor deposition, epitaxial growth, electrochemical deposition, inkjet printing, screen printing, chemical self-assembly, nanoimprint lithography, 3D printing, and two-photon printing.

7. The fluid control device according to any one of claims 1 to 6, wherein a method for fabricating the electrode comprises the following steps:
i) forming grooves on a substrate, wherein a depth of each groove corresponds to the height of the nanochannel;
ii) providing an electrode sacrificial layer on a surface of the substrate, the electrode sacrificial layer having a shape complementary to the intended shape of the electrode; and
iii) depositing an electrode layer on the first substrate coated with the electrode sacrificial layer, and removing the electrode sacrificial layer along with a portion of the electrode structure formed over the electrode sacrificial layer; and obtaining pairs of electrodes disposed within or adjacent to the grooves formed in step i) and extending along an extension direction of the nanochannel; and
iv) setting a thickness of the electrode layer to be 80%-110% of the height of the nanochannel.

8. A method for fabricating the fluid control device according to any one of claims 1 to 7, comprising the following steps:
1) fabricating the nanochannel array, comprising:
1-i) forming grooves on a first substrate, wherein a depth of each groove corresponds to the height of the nanochannel;
1-ii) providing an electrode sacrificial layer on a surface of the first substrate, the electrode sacrificial layer having a shape complementary to the intended shape of the electrode;
1-iii) depositing an electrode layer on the first substrate over the electrode sacrificial layer, and removing the electrode sacrificial layer along with a portion of the electrode structure formed over the electrode sacrificial layer; and obtaining pairs of electrodes disposed within or adjacent to the grooves formed in step 1-i) and extending along an extending direction of the nanochannel;
1-iv) setting a thickness of the electrode layer to be 80%-110% of the height of the nanochannel;
2) fabricating the microchannel array, comprising:
forming the microchannel array on the first substrate, wherein the microchannel array comprises the inlet microchannel array and the outlet microchannel array, and the inlet microchannel array and the outlet microchannel array are in fluid communication through the nanochannel array; or
forming the inlet microchannel array and the outlet microchannel array on a second substrate, wherein the inlet microchannel array comprises at least one inlet microchannel, the outlet microchannel array comprises at least one outlet microchannel, the inlet is in fluid communication with the inlet microchannel, and the outlet is in fluid communication with the outlet microchannel; aligning and bonding at least one first substrate layer comprising the nanochannel array and at least one second substrate layer comprising the microchannel array, wherein the inlet microchannel and the outlet microchannel are in fluid communication through the nanochannel array; providing an outlet and an inlet, wherein the inlet is in fluid communication with the inlet microchannel, and the outlet is in fluid communication with the outlet microchannel; alternately stacking and bonding at least one layer of the first substrate comprising the nanochannel array and at least one layer of the second substrate comprising the microchannel array, wherein the inlet microchannel and the outlet microchannel are in fluid communication only through the nanochannel array, and the outlet and the inlet are disposed on any layer; and
3) forming a micro-nano channel network, the inlet microchannel array and the outlet microchannel array being in fluid communication only through the nanochannel array within the network, all fluid inflow and outflow of the network taking place only via the inlet or the outlet;
wherein preferably, a material of the first substrate is a micro-nano fabrication material, more preferably glass, quartz, a silicon wafer, a silicon nitride wafer, a silicon dioxide wafer, a silicon carbide wafer, polydimethylsiloxane, polymethyl methacrylate, parylene C, polycarbonate, a cyclic olefin copolymer, a cyclic olefin polymer, polypropylene, photocurable resin, or a soft thermoplastic elastomer; and
preferably, a material of the second substrate is a micro-nano fabrication material, more preferably a silicon wafer, polydimethylsiloxane, a silicon nitride wafer, a silicon dioxide wafer, a silicon carbide wafer, glass, quartz, polymethyl methacrylate, parylene C, polycarbonate, a cyclic olefin copolymer, a cyclic olefin polymer, polypropylene, photocurable resin, or a soft thermoplastic elastomer.

9. Use of the fluid control device according to any one of claims 1 to 7, for reversible poration of target biological particles and/or loading of an exogenous substance.
wherein preferably, the biological particles are selected from microparticles having a biological membrane structure,
preferably, the biological particles having a biological membrane structure with a size of 30 nm-2000 nm;
more preferably, the biological particles are selected from extracellular vesicles, membrane vesicles secreted by microorganisms, subcellular particles having membrane structures, cell membrane nanoparticles, artificially synthesized nanoparticles encapsulated by phospholipid bilayer membrane structures, liposomes, or viral vectors.

10. A method for reversible poration of biological particles, the method comprising:
a step of performing reversible poration on target biological particles by using the fluid control device according to any one of claims 1 to 7,
optionally, the loading the exogenous substance into the target biological particles comprises:
mixing the exogenous substance with the target biological particles;
preferably, wherein the mixing is performed before the biological particles are added to the fluid control device, or the mixing is performed within the fluid control device, or the exogenous substance is mixed with the permeabilized biological particles after the biological particles are processed by the fluid control device.
